# EUROPÄISCHE PATENTSCHRIFT

(11) **EP 0 704 428 B1**
(45) Veröffentlichungstag und Bekanntmachung des Hinweises auf die Patenterteilung: **25.02.1998**
(21) Anmeldenummer: 95114708.1
(22) Anmeldetag: 19.09.1995
(51) Int. Cl.: C07C 251/24

(54) **Verfahren zur Herstellung von Salcomin**
Process for the preparation of Salcomin
Procédé pour la préparation de Salcomin

(30) Priorität: 30.09.1994 DE 4435158
(43) Veröffentlichungstag der Anmeldung: 03.04.1996
(73) Patentinhaber: Degussa Aktiengesellschaft, 60311 Frankfurt (DE)
(72) Erfinder: Hübner, Frank, Dr., D-64372 Ober-Ramstadt (DE); Gora, Ulrich, D-63571 Gelnhausen (DE); Huthmacher, Klaus, Dr., D-63571 Gelnhausen (DE); Drauz, Karlheinz, Prof. Dr., D-63579 Freigericht (DE)

(56) Entgegenhaltungen:
- DE-A- 3 302 498
- US-A- 2 508 490
- JOURNAL OF THE AMERICAN CHEMICAL SOCIETY, Bd. 69, 1947 WASHINGTON DC US, Seiten 1886-1893, R.H. BAILES ET AL. 'The Oxygen-carrying Synthetic Chelate Compounds. VII. Preparation'
- BULLETIN DE LA SOCIETE CHIMIQUE DE FRANCE, Nr. 11-12, 1976 PARIS FR, Seiten 1717-1721, D. AYMES ET AL. 'Synthèse de complexes du cobalt(II) avec des base de Schiff: complexes porteurs d'oxygène'
- J. CHEM. ED., Bd. 66, Nr. 10, 1989 Seiten 854-856, D.J. AYMES ET AL. 'Molecular Oxygen Uptake by a solid Co(II)Complex'

## Beschreibung

Die Erfindung betrifft ein Verfahren zur Herstellung von Salcomin, bei dem Ethylendiamin mit etwa der zweifachen molaren Menge Salicylaldehyd, bezogen auf Ethylendiamin, und einem Kobaltsalz unter Verwendung eines organischen Lösungsmittels ohne Zusatz von Wasser im flüssigen Reaktionsmedium bei Temperaturen von 60 - 150 °C umgesetzt wird.

Salcomin, d. h. N,N'-Bis-salicyliden-ethylendiimin-kobalt (II) der Formel 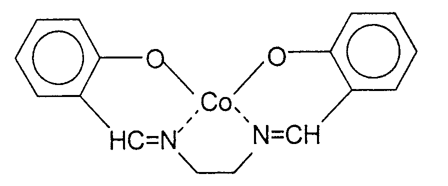 ist ein sehr geeigneter Katalysator für die Oxidation von alkylierten Phenolen zu den entsprechenden alkylierten p-Benzochinonen, insbesondere für die Oxidation von 2,3,6-Trimethylphenol zu 2,3,5-Trimethyl-p-benzochinon, welches leicht z. B. durch Hydrierung in 2,3,5-Trimethylhydrochinon übergeführt werden kann. Dem Trimethylhydrochinon wiederum kommt ein großes Interesse als Vorprodukt für Vitamin E zu.

Bei bekannten Verfahren zur Herstellung von Salcomin verläuft die Reaktion von Ethylendiamin und Salicylaldehyd mit einem Kobalt (II)-Salz beispielsweise in wäßriger oder wäßrig-organischer Lösung.

Es wird auch die Gegenwart eines Puffers (z. B. wäßrige Essigsäure-Natriumacetlösung oder eine Alkanol-Pyridin-Mischung) zum besseren Lösen der Schiffschen Base aus Salicylaldehyd und dem Diamin empfohlen. Das ausfallende Salcomin muß schließlich abfiltriert, mehrfach gewaschen und getrocknet werden.

Nachteilig an diesem sowie anderen bekannten Verfahren , die bei D. Aymes, M. R. Paris, Bull. Soc. Chim. Fr. 1976, 1717 - 21 und D. Aymes, M. R. Paris, J. Chem. Educ., 66 (1989), 854 - 56, zusammengestellt sind, ist vor allem eine aufwendige Aufarbeitung, bei der filtriert, mehrfach gewaschen und getrocknet werden muß.

Darüber hinaus ist das erhaltene Salcomin hinsichtlich seiner Oxidationswirkung nicht immer von einer gleichbleibenden Qualität.

Die von Aymes et al. in zitierter Literatur vorgeschlagenen Verbesserungen, bei der Herstellung von Salcomin als Lösungsmittel Benzol, Chloroform oder Methylenchlorid und das darin lösliche Kobaltacetylacetonat zu verwenden, haben die Nachteile der Verwendung teurer Kobaltverbindungen, niedriger Ausbeuten an Salcomin sowie die Verwendung teilweise gesundheitlich bedenklicher Lösungsmittel.

In der DE-PS 33 02 498 wird ein Verfahren zur Herstellung von Salcomin durch Umsetzen von 2 Mol Salicylaldehyd mit 1 Mol Ethylendiamin und einem Kobaltsalz in flüssigem Reaktionsmedium vorgeschlagen, bei dem man den Salicylaldehyd mit dem Ethylendiamin in einem am Stickstoff substituierten linearen oder cyclischen Carbonsäureamid als Lösungsmittel umsetzt und das erhaltene Reaktionsgemisch bei Temperaturen von 60 bis 150 °C mit Kobaltcarbonat oder dem Kobalthydroxidcarbonat 2 CoCO₃ · 3 Co(OH)₂ umsetzt.

Beispiele für Lösungsmittel gemäß der DE-PS 33 02 498 sind vor allem Dimethylformamid oder N-Methylpyrrolidon. Obwohl in der in Rede stehenden Patentschrift Ausbeuten bezüglich des Salcomins von bis zu 99 % berichtet werden, gelang es nicht, diese hohen Ausbeuten zu verifizieren. Entgegen den Angaben der Patentschrift lagen in Vergleichsversuchen die Ausbeuten nur bei ca. 60 % für Kobalthydroxidcarbonat (siehe hierin unter Vergleichsbeispiel 1 ≅ Beispiel 3 aus DE-PS 33 02 498) oder bei ca. 90 % bei Verwendung von Kobaltcarbonat (Beispiel 1 aus DE-PS 33 02 498), wobei außerdem beim Verfahren gemäß der DE-PS 33 02 498 die Verwendung von gesundheitsschädigenden Lösungsmitteln bedenklich erscheint.

Verfahren zur Herstellung von dem Salcomin ähnlichen chiralen Katalysatoren sind aus der WO 93/03838 bekannt. Allerdings werden dort ausschließlich 3-wertige Metallkomplexe offenbart.

Eine dem Salcomin ähnliche Verbindung beschreibt auch die DE-A-42 38 076. Allerdings ist das Löslichkeitsverhalten von Co(5-NO₂-Saltmen) deutlich von dem des Salcomins verschieden.

R.H. Bailes und M. Calvin (J. Am.Chem.Soc. 1947, Vol. 69, 1886-1893) beschreiben ein Verfahren zur Herstellung von Salcomin, bei dem dem Reaktionsgemisch neben dem organischen Lösungsmittel Wasser zugesetzt wird.
Die reaktive Form des Salcomins erreicht man nach dieser Vorschrift nur über die Form des Pyridinats.

Angesichts des hierin diskutierten Standes der Technik ist es mithin Aufgabe der Erfindung, ein weiteres Verfahren zur Herstellung von Salcomin anzugeben, das möglichst weitgehend auf die Verwendung schädlicher oder gesundheitlich bedenklicher Lösungsmittel verzichtet und gleichzeitig eine möglichst hohe, am besten genauso gute oder bessere Salcominausbeute ermöglicht, als sie von den Verfahren nach dem Stand der Technik bekannt ist.

Gelöst werden diese und weitere nicht im einzelnen aufgeführte Aufgaben durch ein Verfahren der eingangs erwähnten Gattung mit den Merkmalen des kennzeichnenden Teils des Anspruchs 1.

Vorteilhafte Varianten des erfindungsgemäßen Verfahrens werden in den auf Anspruch 1 rückbezogenen Ansprüchen unter Schutz gestellt.

Dadurch, daß man als Lösungsmittel einen oder mehrere aliphatische und/oder cycloaliphatische Alkohole R-OH, worin R für einen linearen, verzweigten oder cyclischen C₃-C₁₂-Alkylrest steht, und/oder einen oder mehrere aromatische Kohlenwasserstoffe mit 6 - 15 Kohlenstoffatomen verwendet und als Kobaltsalz Kobaltcarbonat, basisches Kobaltcarbonat (CoCO₃ · Co(OH)₂) und/oder Kobaltacetat, ergeben sich bei der erfindungsgemäßen Herstellung von Salcomin erhebliche Vorteile gegenüber dem Stand der Technik; hierzu gehören u. a.:
A) Eine Verwendung des giftigen Lösungsmittels Dimethylformamid ist nicht notwendig.
B) Die Ausbeuten an Salcomin liegen bei über 90 - 99 % gegenüber ca. 60 % - 90 % nach dem Stand der Technik (siehe Vergleichsbeispiele 1 und 1a).
C) Das lösungsmittelfeuchte Salcomin kann ohne weitere Trocknung zur Oxidation von Trimethylphenol eingesetzt werden.
D) Die Ausbeute an Trimethyl-p-benzochinon liegt meist bei über 90 %, gleichgültig, ob von getrockneten oder feuchten Katalysatoren ausgegangen wird.
E) Das Reaktionswasser kann leicht azeotrop ausgekreist werden.
F) Das Salcomin kristallisiert analysenrein aus der Mischung aus.
G) Das hergestellte Salcomin ist lagerstabil.

Dabei ist die einfache erfindungsgemäße Herstellung von Salcomin in aromatischen Kohlenwasserstoffen bzw. aliphatischen Alkoholen unter Verwendung der in diesen Lösungsmitteln im wesentlichen unlöslichen Kobaltsalzen, wie Kobaltcarbonat, basisches Kobaltcarbonat oder Kobaltacetat , sehr überraschend, da aus dem dargelegten Stand der Technik hervorgeht, daß das verwendete Kobaltsalz in dem für die Umsetzung verwendeten Lösungsmittel löslich sein muß, oder bei Verwendung von Kobaltcarbonat bzw. basischem Kobaltcarbonat das Lösungsmittel Dimethylformamid oder ein entsprechendes am Stickstoff disubstituiertes lineares oder cyclisches Carbonsäureamid sein muß.

Demgegenüber hat die Erfindung in nicht ohne weiteres vorhersehbarer Weise ergeben, daß die Umsetzung von Salicylaldehyd mit Ethylendiamin mit einem oder mehreren Kobaltsalzen aus der Gruppe Kobaltcarbonat, basisches Kobaltcarbonat und Kobaltacetat auch unter Verwendung von anderen organischen Lösungsmitteln als DMF oder N-Methylpyrrolidon zu hervorragenden Ergebnissen bezüglich Ausbeute an Salcomin sowie dessen Güte als Oxidationskatalysator führt.

Zu den erfindungsgemäß einzusetzenden Lösungsmitteln gehören grundsätzlich die dem Fachmann geläufigen aliphatischen und cycloaliphatischen Alkohole mit 3 bis 12 Kohlenstoffatomen sowie aromatische Kohlenwasserstoffe mit 6 bis 15 Kohlenstoffatomen.

Bevorzugte aromatische Lösungsmittel zur erfindungsgemäßen Verwendung sind Toluol, Xylole oder andere mit Alkylgruppen substituierte Aromaten der Formeln I - III 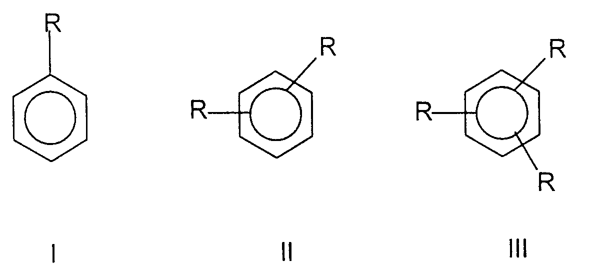 wobei R Alkylgruppen mit bis zu 6 C-Atomen bedeuten, bei einer Mehrfach-Substitution jedoch die Anzahl C-Atome aller Reste auf zusammen 9 begrenzt ist.

Erfindungsgemäße aromatische Kohlenwasserstoffe mit 6 bis 15 C-Atomen sind u. a. Toluol, Cumol, Xylol und Mesitylen.

Aliphatische bzw. cycloaliphatische Alkohole als Lösungsmittel können Verbindungen der Formeln IV bzw. V sein,

R―OH IV

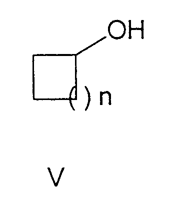

wobei R n-Alkyl von 3 - 12 Kohlenstoffatomen bzw. verzweigte Alkylgruppen von 3 - 12 Kohlenstoffatomen sein können.

Hierzu gehören u. a. n-Propanol, iso-Propanol, n-Butanol, sec.-Butanol, tert.-Butanol, n-Pentanol, iso-Amylalkohol, n-Hexanol.

Bei cycloaliphatischen Alkoholen der Formel V kann n eine Zahl von 0 - 9 sein. Beispielhafte cycloaliphatische Alkohole umfassen u. a. Cyclobutanol, Cyclopentanol, Cyclohexanol, Cycloheptane und Cyclooctanol.

Die vorgenannten Lösungsmittel werden entweder allein oder in Kombination von zwei oder mehreren verwendet.

Für die Erfindung besonders bevorzugte Lösungsmittel sind n-Butanol oder Toluol, weil hierin besonders hohe Ausbeuten an Salcomin erhalten werden und die betreffenden Lösungsmittel relativ unbedenklich und recht preisgünstig sind.

Unter den für die Erfindung einzusetzenden Salzen sind vor allem Kobaltcarbonat und/oder basisches Kobaltcarbonat zu nennen. Erstaunlicherweise haben sich auch unter Verwendung von Kobaltacetat Co(OAc)₂ · 4 H₂O in n-Butanol sehr hohe Ausbeuten gezeigt. Insbesondere zeigte ein derart hergestelltes Salcomin einen hervorragenden Umsatz in der Oxidationsreaktion von Trimethylphenol zu Trimethyl-p-benzochinon.

Dieser Befund steht wiederum im deutlichen Gegensatz zum Stand der Technik, in dem die Meinung vertreten wurde, daß die Reaktionsgeschwindigkeiten für die Trimethylphenol-Oxidation mit lösungsmittelfeuchtem Salcomin bei aus Co(OAc)₂ hergestellten Salcominen um etwa 10 bis 15 % geringer waren und nur das aus CoCO₃ oder aus Kobalthydroxidcarbonat hergestellte Salcomin einen unverändert guten Umsatz zeigte.

In bevorzugter erfindungsgemäßer Verfahrensabwandlung wird die Umsetzung von Salicylaldehyd, Ethylendiamin und Kobaltsalz unter "Auskreisen" des bei der Reaktion entstehenden Wassers, der bei der Reaktion ggf. entstehenden Essigsäure sowie des im eingesetzten Kobaltsalz enthaltenen Wassers durchgeführt. Die "Auskreisung", also Entfernung, aus dem Reaktionsgemisch erfolgt am zweckmäßigsten azeotrop.

Besonders vorteilhaft kann die erfindungsgemäße Herstellung von Salcomin dadurch gestaltet werden, daß die Umsetzung unter Inertgasatmosphäre, also z. B. unter Stickstoff oder unter Argon durchgeführt wird.

Zur Durchführung des erfindungsgemäßen Verfahrens zur Herstellung von Salcomin werden beispielsweise 2 Mol Salicylaldehyd und 1 Mol Ethylendiamin im aromatischen Kohlenwasserstoff bzw. dem aliphatischen Alkohol unter Stickstoff bei ca. 60 °C gelöst und nach Zugabe von 1 Mol Kobaltcarbonat oder basischem Kobaltcarbonat ca. 1 h (0,5 - 2 h) auf Siedetemperatur erhitzt. Nach Abscheiden von ca. 1 Mol Wasser ist die Reaktion beendet und das Salcomin kristallisiert quantitativ aus.

Die Erfindung wird im weiteren anhand von Ausführungsbeispielen weiter erläutert.

### Vergleichsbeispiel 1:

### Salcominherstellung nach Angaben der PS 33 02 498 (Beispiel 3)

244 g (2 Mol) Salicylaldehyd wurden in 700 ml DMF gelöst und innerhalb von 30 min mit 60 g (1 Mol) Ethylendiamin versetzt. Dabei stieg die Temperatur auf 60 °C an und ein gelber kristalliner Feststoff fiel aus. Anschließend wurde auf 120 °C erhitzt und portionsweise innerhalb 30 min mit 125 g (1 Mol Kobalt) CoCO₃ · Co(OH)₂ (Kobaltgehalt > 47 %) versetzt. Nach einer Stunde Nachreaktion bei 120 °C unter leichter Gasentwicklung wurde auf Raumtemperatur (RT) abgekühlt und der kristalline Niederschlag abfiltriert. Die Ausbeute an getrocknetem Salcomin betrug 204 g entsprechend 63 % der Theorie.

| | | | | |
|---|---|---|---|---|
| CH-Analyse: | Ber. | C 59.09 | H 4,34 | N 8,61 |
| | Gef. | C 52,20 | H 3,82 | N 7,50 |

### Vergleichsbeispiel 1a:

Wie Vergleichsbeispiel 1, es wurde jedoch die entsprechende Menge Kobaltcarbonat eingesetzt. Die Ausbeute lag bei 90 % d. Th.

### Beispiel 2

Unter Stickstoff wurden 12,2 g (0,1 Mol) Salicylaldehyd in 125 ml n-Butanol gelöst und innerhalb 15 min 3,0 g (0,05 Mol) Ethylendiamin bei 50 °C zugetropft. Anschließend wurde weitere 30 min bei 60 °C gerührt. Man erhielt eine gelbe Suspension mit kristallinem Feststoff. Nach Abkühlen auf RT wurden 6,25 g (0,05 Mol) basisches Kobaltcarbonat oder 6,4 g (0,05 Mol) Kobaltcarbonat (> 46 % Kobalt) zugesetzt und unter fortgesetztem Einleiten von Stickstoff 1,5 h zum Sieden erhitzt. Dabei wurden etwa 40 ml n-Butanol und 2 ml Wasser abdestilliert. Nach Abkühlung auf RT wird der kristalline Niederschlag abgesaugt. Ausbeute: 16,1 g getrocknetes Salcomin entsprechend 99 % der Theorie.

| | | | | |
|---|---|---|---|---|
| CH-Analyse: | Ber. | C 59,09 | H 4,34 | N 8,61 |
| | Gef. | C 59,10 | H 4,41 | N 8,70 |

### Beispiel 3

Es wurde wie in Beispiel 2 verfahren, jedoch wurde anstelle von n-Butanol als Lösungsmittel 125 ml Toluol verwendet. Man erhielt 14,5 g getrocknetes Salcomin entsprechend 89 % der Theorie.

| | | | | |
|---|---|---|---|---|
| CH-Analyse: | Ber. | C 59,09 | H 4,34 | N 8,61 |
| | Gef. | C 58,27 | H 4,28 | N 8,45 |

### Beispiel 4:

Es wurde wie im Beispiel 2 verfahren, jedoch wurde anstelle von basischem Kobaltcarbonat mit 12,5 g (0,05 Mol) Kobaltacetat Co(OAc)₂ · 4 H₂O umgesetzt und ca. 2,5 ml Wasser abgeschieden. Man erhielt 16,1 g (99 % der Theorie) getrocknetes Salcomin.

| | | | |
|---|---|---|---|
| Ber. | C 59,09 | H 4,34 | N 8,61 |
| | C 57,65 | H 4,35 | N 8,24 |

### Beispiel 5

Es wurde wie im Beispiel 1 verfahren, jedoch wurde während der gesamten Operation unter Stickstoff gearbeitet. Man erhielt 13,7 g (84 % der Theorie) getrocknetes Salcomin.

| | | | |
|---|---|---|---|
| Ber. | C 59,09 | H 4,34 | N 8,61 |
| Gef. | C 58,94 | H 4,38 | N 8,72 |

### Vergleichsbeispiel 6 und Beispiele 7 - 10:

### Oxidation von 2,3,6-Trimethylphenol (TMP) zu 2,3,5-Trimethyl-p-benzochinon (TMC)

Die in Beispiel 1 - 5 hergestellten Salcomine wurden in der Oxidationsreaktion von TMP zu TMC untersucht. Dazu wurden 20 g TMP in 59 g (62 ml) DMF gelöst und mit je 0,6 g (berechnet auf trockenes Salcomin) Salcomin der Beispiele 1 - 5 versetzt. Anschließend wurde mit Sauerstoff zunächst 4 h bei 30 °C und dann 2 weitere Stunden bei 42 °C oxidiert. Der Gehalt an Trimethyl-p-benzochinon (TMC) in der Lösung wurde durch HPLC bestimmt.

**Tabelle**

| **Beispiel/Vergleichsbeispiel** | **Salcomin aus Beispiel** | **Co-Salz** | **Lösungsmittel d. Salcomin- Herstellung** | **Ausbeute TMC (% d. Theorie) Salcomin** | |
|---|---|---|---|---|---|
| | | | | **feucht** | **getrocknet** |
| 6 | 1 (Vgl. Stand der Technik) | CoCO₃ · Co(OH)₂ | DMF | 90,6 | 90,8 |
| 7 | 2 | CoCO₃ und | n-Butanol | 91,8 | 92,4 |
| | | CoCO₃ · Co(OH)₂ | | 90,6 | 90,8 |
| 8 | 3 | CoCO₃ · Co(OH)₂ | Toluol | 91,9 | 91,9 |
| 9 | 4 | Co(OAc)₂ · 4 H₂O | n-Butanol | 91,8 | 92,2 |
| 10 | 5 | CoCO₃ · Co(OH)₂ | DMF | 91,6 | 91,9 |

Weitere Vorteile und Ausführungsformen ergeben sich aus den folgenden Patentansprüchen.

## Patentansprüche

1. Verfahren zur Herstellung von Salcomin,
bei dem Ethylendiamin mit etwa der zweifachen molaren Menge Salicylaldehyd, bezogen auf Ethylendiamin, und einem Kobaltsalz unter Verwendung eines organischen Lösungsmittels ohne Zusatz von Wasser im flüssigen Reaktionsmedium bei Temperaturen von 60 - 150 °C umgesetzt wird,
**dadurch gekennzeichnet,**
daß man als Lösungsmittel
einen oder mehrere aliphatische und/oder cycloaliphatische Alkohole R-OH, worin R für einen linearen, verzweigten oder cyclischen C₃-C₁₂-Alkylrest steht, und/oder einen oder mehrere aromatische Kohlenwasserstoffe mit 6 - 15 Kohlenstoffatomen verwendet
und als Kobaltsalz
Kobaltcarbonat, basisches Kobaltcarbonat (CoCO₃ · Co(OH)₂) und/oder Kobaltacetat.

2. Verfahren nach Anspruch 1,
**dadurch gekennzeichnet,**
daß man n-Butanol als Lösungsmittel verwendet.

3. Verfahren nach Anspruch 1 oder 2,
**dadurch gekennzeichnet,**
daß man Kobaltcarbonat und/oder basisches Kobaltcarbonat als Kobaltsalz verwendet.

4. Verfahren nach Anspruch 1,
**dadurch gekennzeichnet,**
daß man Toluol als Lösungsmittel verwendet.

5. Verfahren nach einem der vorhergehenden Ansprüche,
**dadurch gekennzeichnet,**
daß man die Umsetzung unter Auskreisen des bei der Reaktion entstehenden Wassers oder Essigsäure sowie des im eingesetzten Kobaltsalz enthaltenen Wassers durchführt.

6. Verfahren nach einem der vorhergehenden Ansprüche,
**dadurch gekennzeichnet,**
daß man die Umsetzung unter einem Schutzgas, bevorzugt Stickstoff, durchführt.

## Claims

1. A process for the preparation of salcomin, wherein ethylenediamine is reacted in a liquid reaction medium at temperatures of from 60°C to 150°C with approximately twice the molar quantity of salicylaldehyde, referred to ethylenediamine, and with a cobalt salt, with the use of an organic solvent without the addition of water,
characterised in that
one or more aliphatic and/or cycloaliphatic alcohols R-OH, wherein R represents a linear, branched or cyclic C₃-C₁₂ alkyl group, and/or one or more aromatic hydrocarbons having 6 to 15 carbon atoms are used as solvent
and cobalt carbonate, basic cobalt carbonate (CoCO₃ · Co(OH)₂) and/or cobalt acetate are used as the cobalt salt.

2. A process according to claim 1,
characterised in that
n-butanol is used as solvent.

3. A process according to claim 1 or 2,
characterised in that
cobalt carbonate and/or basic cobalt carbonate are used as the cobalt salt.

4. A process according to claim 1,
characterised in that
toluene is used as solvent.

5. A process according to one of the preceding claims,
characterised in that
the reaction is carried out accompanied by removal of the water or acetic acid formed during the reaction and of the water contained in the cobalt salt used.

6. A process according to one of the preceding claims,
characterised in that
the reaction is carried out under a protective gas, preferably nitrogen.

## Revendications

1. Procédé pour la préparation de salcomine, dans lequel on fait réagir de l'éthylènediamine avec approximativement deux fois la quantité molaire de salicylaldéhyde rapportée à l'éthylènediamine et un sel de cobalt en utilisant un solvant organique, sans addition d'eau, dans un milieu de réaction liquide, à des températures de 60 à 150°C, caractérisé en ce qu'on utilise, à titre de solvant, un ou plusieurs alcools aliphatiques et/ou cycloaliphatiques R-OH où R représente un radical alkyle en C₃-C₁₂ linéaire, ramifié ou cyclique et/ou un ou plusieurs hydrocarbures aromatiques contenant de 6 à 15 atomes de carbone et, à titre de sel de cobalt, du carbonate de cobalt, du carbonate de cobalt basique (CoCO₃ · Co(OH)₂ et/ou de l'acétate de cobalt.

2. Procedé selon la revendication 1, caractérisé en ce qu'on utilise du n-butanol comme solvant.

3. Procédé selon la revendication 1 ou 2, caractérisé en ce qu'on utilise du carbonate de cobalt et/ou du carbonate de cobalt basique comme sel de cobalt.

4. Procédé selon la revendication 1, caractérisé en ce qu'on utilise du toluène comme solvant.

5. Procédé selon l'une quelconque des revendications précédentes, caractérisé en ce qu'on effectue la mise an réaction en évacuant l'eau ou l'acide acétique en circulation que l'on obtient au cours de la réaction, ainsi que l'eau contenue dans le sel de cobalt mis en oeuvre.

6. Procédé selon l'une quelconque des revendications précédentes, caractérisé en ce qu'on effectue la mise en réaction sous l'atmosphère d'un gaz de protection, de préférence sous atmosphère d'azote.
